# EUROPÄISCHE PATENTSCHRIFT

(11) **EP 0 996 605 B1**
(45) Veröffentlichungstag und Bekanntmachung des Hinweises auf die Patenterteilung: **29.01.2003**
(21) Anmeldenummer: 98936425.2
(22) Anmeldetag: 15.07.1998
(51) Int. Cl.: C07B 31/00, C07C 29/141, C07C 31/12, C07C 31/22, B01J 25/00

(54) **Verfahren zur Hydrierung von Carbonylverbindungen**
Method for the hydrogenation of carbonyl compounds
Procédé pour l'hydrogenation de composés carbonyles

(30) Priorität: 18.07.1997 DE 19730939
(43) Veröffentlichungstag der Anmeldung: 03.05.2000
(73) Patentinhaber: BASF AKTIENGESELLSCHAFT, 67056 Ludwigshafen (DE)
(72) Erfinder: BREITSCHEIDEL, Boris, D-67117 Limburgerhof (DE); WALTER, Marc, D-67227 Frankenthal (DE); KRATZ, Detlef, D-69121 Heidelberg (DE); SCHULZ, Gerhard, D-67069 Ludwigshafen (DE); SAUERWALD, Manfred, D-67149 Meckenheim (DE)
(74) Vertreter: Isenbruck, Günter, Dr.
(86) Internationale Anmeldenummer: EP9804402
(87) Internationale Veröffentlichungsnummer: WO99003801

(56) Entgegenhaltungen:
- WO-A-95/32171
- RO-B- 106 741
- DATABASE WPI Section Ch, Week 9434 Derwent Publications Ltd., London, GB; Class E17, AN 94-277308 XP002080579 & RO 106 741 B (COMB CHIM VICTORIA) , 30. Juni 1993 in der Anmeldung erwähnt
- PATENT ABSTRACTS OF JAPAN vol. 015, no. 357 (C-0866), 10. September 1991 & JP 03 141235 A (MITSUI PETROCHEMICAL INDUSTRY), 17. Juni 1991

## Beschreibung

Die Erfindung betrifft ein Verfahren zur katalytischen Hydrierung von Carbonylverbindungen in Gegenwart eines Raney-Kupfer-Katalysators.

Die katalytische Hydrierung von Carbonylverbindungen, wie z. B. die Hydrierung von Aldehyden zur Herstellung einfacher und funktionalisierter Alkohole nimmt in den Produktionssträngen der chemischen Grundstoffindustrie eine bedeutende Stellung ein. Besonders gilt dies für die Hydrierung von durch die Oxosynthese oder durch Aldolreaktion zugänglichen Aldehyden.

Die katalytische Hydrierung von Aldehyden in Suspensions- oder Festbettfahrweise ist seit langem bekannt. Technische Anlagen arbeiten fast ausschließlich mit Festbettreaktoren.

Als Festbettkatalysatoren finden vor allem Trägerkatalysatoren, beispielsweise mit SiO₂- oder Al₂O₃ geträgerte Cu/Ni- oder Cu/Cr-Katalysatoren Verwendung.

Als Alternative zu den Trägerkatalysatoren bieten sich Katalysatoren vom Raney-Typ an. Raney-Katalysatoren zeigen, bedingt durch die große Metall-Oberfläche, eine besonders hohe Hydrieraktivität. Als Metalle kommen Nickel, Cobalt, Eisen und Kupfer in Frage.

In der SU 430 876 wird die Hydrierung von Furfural an einem Raney-Kupfer-Katalysator in Suspensionsfahrweise beschrieben. Suspensionsverfahren haben gegenüber Festbettverfahren den Nachteil eines höheren Katalysatorverbrauchs. Ferner muß der Katalysator in einem zusätzlichen Verfahrensschritt vom Reaktionsgemisch abgetrennt werden.

In der RO 106 741 ist ein Verfahren zur Hydrierung von Furfural zu Furfurylalkohol in einem Festbettreaktor in Rieselfahrweise beschrieben. Unter anderem wird als Katalysator Raney-Kupfer eingesetzt. Über die Herstellung des Katalysators und seine Eigenschaften enthält die Schrift keine Angaben.

Die JP-A-3 141 235 beschreibt ein Verfahren zur Hydrierung von Aceton zu Isopropanol, wobei ein Raney-Nickel-Katalysator eingesetzt wird. Es ist zwar beschrieben, daß der Raney-Ni-Katalysator mit einem Raney-Cu-Katalysator gemischt werden kann. Gleichzeitig wird jedoch impliziert gesagt, daß die Beimischung ab einer bestimmten Menge zu einer Produktivitäterniedrigung führt.

Üblicherweise werden im Festbettverfahren verwendbare Raney-Katalysatoren hergestellt. indem eine pulverförmige Aluminium-Kupfer-Legierung mit Binde- und Hilfsmitteln verknetet, aus der Knetmasse Formkörper, beispielsweise Tabletten oder Stränge hergestellt, die Formkörper calciniert und die calcinierten Formkörper durch Behandlung mit einem Alkalimetallhydroxid aktiviert werden. Solche Herstellverfahren für Raney-Katalysatoren sind beispielsweise in DE-A 43 45 265, Ind. Eng. Chem. Res. 28 (1989), S. 1764 - 1767 und in DE-A 44 46 907 beschrieben. Die Herstellung des Katalysators nach diesen Verfahren umfaßt mehrere Verfahrensschritte.

Der Erfindung lag die Aufgabe zugrunde, ein Verfahren zur katalytischen Hydrierung von Carbonylverbindungen unter Einsatz eines technisch einfach herzustellenden Katalysators, der eine hohe Aktivität und Selektivität aufweist, bereitzustellen.

Es wurde überraschenderweise gefunden, daß Raney-Kupfer, das in Form sogenannter Nuggets eingesetzt wird, für die Hydrierung von Carbonylverbindungen zu, z. B. den entsprechenden Alkoholen eine hohe katalytische

Aktivität und Selektivität aufweist, welche die Aktivität und Selektivität von nach dem Stand der Technik hergestellten Raney-Kupfer-Katalysatoren übertrifft.

Demgemäß wurde die Aufgabe gelöst durch ein Verfahren zur katalytischen Hydrierung einer Carbonylverbindung oder eines Gemisches aus zwei oder mehr Carbonylverbindungen in Gegenwart eines Raney-Kupfer-Katalysators, das dadurch gekennzeichnet ist, daß der Raney-Kupfer-Katalysator in Form von Nuggets eingesetzt wird.

Unter Nuggets soll grobkörniges Metall aus Körnern unregelmäßiger Geometrie mit einer Korngröße von 0,5 bis 10 mm verstanden werden. Die Raney-Kupfer-Nuggets werden aus grobkörniger Kupfer-Aluminium-Legierung ohne die Zwischenschritte des Verknetens der Kupfer-Aluminium-Legierung mit einem Binde- und/oder Hilfsmittel, Formen der Knetmasse zu Formkörpern und Calcinieren der Formkörper, durch Behandeln der grobkörnigen Kupfer-Aluminium-Legierung mit Alkalimetallhydroxid hergestellt.

Die Korngröße der in dem erfindungsgemäßen Verfahren eingesetzten Nuggets kann 0,5 bis 10 mm betragen. Bevorzugt liegt sie von 1 bis 8 mm, besonders bevorzugt von 2 bis 7 mm, insbesondere von 2 bis 6 mm.

Bei der Herstellung des in dem erfindungsgemäßen Verfahren verwendeten Raney-Kupfer wird von einer Kupfer-Aluminium-Legierung ausgegangen. Die Herstellung der Kupfer-Aluminium-Legierung erfolgt in an sich bekannter Weise, beispielsweise nach dem in DE-A 21 59 736 beschriebenen Verfahren. Das Massenverhältnis von Aluminium zu Kupfer in der Ausgangslegierung wählt man in der Regel im Bereich von 30 : 70 bis 70 : 30 Gew.-%, bevorzugt von 40 : 60 bis 60 : 40 Gew.-%.

Aus der Kupfer-Aluminium-Legierung wird der Raney-Kupfer-Katalysator durch Herauslösen der katalytisch inaktiven Bestandteile mit Alkalimetallhydroxid (Aktivierung) hergestellt. Bevorzugte Alkalimetallhydroxide sind Natriumhydroxid oder Kaliumhydroxid, besonders bevorzugt ist Natriumhydroxid. In der Regel setzt man eine wäßrige Lösung des Alkalimetallhydroxids, bevorzugt Natronlauge oder Kalilauge, besonders bevorzugt Natronlauge ein, wobei man üblicherweise eine 5 bis 30 gew.-% ige wäßrige Lösung des Alkalimetallhydroxids verwendet. Das Molverhältnis von Alkalimetallhydroxid zu Aluminium wählt man in der Regel im Bereich von 1 : 1 bis 4 : 1, vorzugsweise von 1,5 : 1 bis 2,5 : 1. Die Temperatur der Aktivierung wählt man üblicherweise im Bereich von 25°C bis 95°C, vorzugsweise 45 bis 90 °C. Die Dauer der Aktivierung hängt im wesentlichen vom gewünschten Endgehalt an Aluminium ab und liegt üblicherweise im Bereich von 10 bis 30, bevorzugt von 15 bis 25 h. Zweckmäßig kontrolliert man die Aktivierung durch Messung der bei der Aktivierung freigesetzten Wasserstoffmenge. Der Aktivierungsvorgang kann auch mehrmals durchgeführt werden.

Ausgangsmaterial der Aktivierung ist üblicherweise die grobkörnige Kupfer-Aluminium-Legierung. Die Korngröße der grobkörnigen Kupfer-Aluminium-Legierung kann der Korngröße der in dem erfindungsgemäßen Verfahren eingesetzten Raney-Kupfer-Nuggets entsprechen. Es kann aber auch nach der Aktivierung eine Nachzerkleinerung auf die gewünschte Korngröße stattfinden.

Die in dem erfindungsgemäßen Verfahren eingesetzten Raney-Kupfer-Katalysatoren weisen vorzugsweise einen Kupfer-Gehalt von 40 bis 90 Gew.-%, weiter bevorzugt 50 bis 80 Gew.-%, besonders bevorzugt 60 bis 70 Gew.-% auf.

Die spezifische Oberfläche nach Langmuir in dem erfindungsgemäßen Verfahren eingesetzten Raney-Kupfer-Katalysatoren beträgt vorzugsweise 5 bis 50 m²/g, weiter bevorzugt 15 bis 40 m²/g, besonders bevorzugt von 20 bis 40 m²/g. Die Oberfläche nach Langmuir wird durch Stickstoffabsorption nach DIN 66 132 bestimmt.

Eine charakteristische Größe der erfindungsgemäßen Raney-Kupfer-Katalysatoren ist auch deren spezifische Cu-Oberfläche (S-Cu). Sie wird durch Oxidation von Oberflächen-Kupfer-Atomen mit gasförmigem N₂O in einer erhitzten Probe des Katalysators aus dem ermittelten N₂O-Verbrauch berechnet.

Dazu wird die Probe zunächst 4 Stunden lang mit 10 mbar H₂ bei einer Temperatur von 240 °C behandelt. Anschließend wird die Probe bis zu einem Druck von kleiner als 10⁻³ mbar evakuiert und danach 3 Stunden lang mit 30 mbar H₂ behandelt, anschließend nochmals auf kleiner 10⁻³ mbar evakuiert, 3 Stunden lang mit 100 mbar H₂ behandelt, wiederum auf kleiner 10⁻³ mbar evakuiert und abschließend nochmals 3 Stunden lang mit 200 mbar H₂ behandelt, wobei die Behandlung mit Wasserstoff jeweils bei einer Temperatur von 240°C durchgeführt wurde.

In einer zweiten Stufe wird die Probe mit N₂O bei einer Temperatur von 70 °C bei einem Druck von 266 mbar 2 Stunden lang beaufschlagt, wobei eine Zersetzung des N₂O an der Probe zu beobachten ist; anschließend wird die Probe auf kleiner 10⁻³ mbar evakuiert und danach die Massezunahme des Katalysators infolge der Bildung von Kupferoxid an der Oberfläche desselben bestimmt.

Die spezifische Cu-Oberfläche der Raney-Kupfer-Katalysatoren beträgt vorzugsweise von 0,5 bis 7 m²/g, weiter bevorzugt liegt sie von 1 bis 4 m²/g.

Das durch Quecksilber-Porosimetrie ermittelte Porenvolumen des Raney-Kupfer-Katalysators beträgt vorzugsweise 0,01 bis 0,12 ml/g, weiter bevorzugt beträgt es 0,03 bis 0,08 ml/g. Der nach dieser Methode ermittelte mittlere Porendurchmesser beträgt vorzugsweise 50 bis 300 nm, weiter bevorzugt 60 bis 100 nm. Das Quecksilber-Porenvolumen und der Porendurchmesser werden gemäß DIN 66 133 bestimmt.

Die Schüttdichte der in dem erfindungsgemäßen Verfahren eingesetzten Nuggets aus Raney-Kupfer beträgt im allgemeinen von 1,9 bis 2,4, bevorzugt von 1,9 bis 2,1 g/ml.

Das erfindungsgemäße Verfahren kann als Festbettreaktion mit fest angeordnetem Katalysator oder als Wirbelbettreaktion mit in auf- und abwirbelnder Bewegung befindlichem Katalysator durchgeführt werden. Bevorzugt ist das Arbeiten im Festbett. Die Hydrierung kann in der Gasphase oder in Flüssigphase durchgeführt werden. Vorzugsweise wird die Hydrierung in flüssiger Phase durchgeführt, beispielsweise in Riesel- oder Sumpffahrweise.

In einer bevorzugten Ausführungsform des erfindungsgemäßen Verfahrens in Rieselfahrweise wird ein Teil des Produkts nach Durchgang durch den Reaktor als Produktstrom kontinuierlich abgezogen, der andere Teil des Produkts zusammen mit frischem, die Carbonylverbindung enthaltendem Edukt dem Reaktor erneut zugeführt. Diese Verfahrensweise wird im folgenden als Kreislauffahrweise bezeichnet.

Bei Arbeiten in Rieselfahrweise läßt man das flüssige, die zu hydrierende Carbonylverbindung enthaltende Edukt in dem Reaktor, der unter einem hohen Wasserstoffdruck steht, über das in diesem angeordnete Katalysatorbett rieseln, wobei sich auf dem Katalysator ein dünner Flüssigkeitsfilm ausbildet. Dagegen wird beim Arbeiten in Sumpffahrweise Wasserstoffgas in den mit der flüssigen Reaktionsmischung gefluteten Reaktor eingeleitet, wobei der Wasserstoff das Katalysatorbett in aufsteigenden Gasperlen passiert.

In Sumpffahrweise kann das erfindungsgemäße Verfahren sowohl diskontinuierlich als auch kontinuierlich durchgeführt werden. Kontinuierlich kann das Verfahren so durchgeführt werden, daß das flüssige Produkt nach Durchgang durch den Reaktor vollständig abgezogen oder aber, analog zu der oben geschilderten Verfahrensweise, nur ein Teil des Produkts abgezogen und der andere Teil des Produkts zusammen mit frischem, die Carbonylverbindung enthaltendem Edukt in den Reaktor zurückgeführt wird (Kreislauffahrweise). Bevorzugt wird das Verfahren kontinuierlich durchgeführt, wobei das gesamte Produkt nach einmaligem (geraden) Durchgang des Edukts durch den Reaktor abgezogen wird.

Das erfindungsgemäße Verfahren eignet sich zur Hydrierung von Carbonylverbindungen wie z.B. Aldehyden und Ketonen zu den entsprechenden Alkoholen, wobei aliphatische und cycloaliphatische gesättigte und ungesättigte Carbonylverbindungen bevorzugt sind. Bei aromatischen Carbonylverbindungen kann es zur Bildung unerwünschter Nebenprodukte durch Hydrierung des aromatischen Kerns kommen. Die Carbonylverbindungen können weitere funktionelle Gruppen wie Hydroxy- oder Aminogruppen tragen. Ungesättigte Carbonylverbindungen werden in der Regel zu den entsprechenden gesättigten Alkoholen hydriert. Der Begriff "Carbonylverbindungen" wie er im Rahmen der Erfindung verwendet wird, umfaßt alle Verbindungen, die eine C=O-Gruppe aufweisen, einschließlich Carbonsäuren und deren Derivaten.

Bevorzugt wird das erfindungsgemäße Verfahren zur Hydrierung aliphatischer Aldehyde, Hydroxyaldehyde, Ketone, Säuren, Ester, Anhydride, Lactone und Zucker eingesetzt.

Bevorzugte aliphatische Aldehyde sind verzweigte und unverzweigte gesättigte und/oder ungesättigte aliphatische C₂-C₃₀-Aldehyde, wie sie beispielsweise durch Oxosynthese aus linearen oder verzweigten Olefinen mit interner oder terminaler Doppelbindung erhältlich sind.

Als Beispiel für aliphatische Aldehyde sind zu nennen:
Propionaldehyd, n-Butyraldehyd, iso-Butyraldehyd, Valeraldehyd, 2-Methylbutyraldehyd, 3-Methylbutyraldehyd (Isovaleraldehyd), 2,2-Dimethylpropionaldehyd (Pivalinaldehyd), Capronaldehyd, 2-Methylvaleraldehyd, 3-Methylvaleraldehyd, 4-Methylvaleraldehyd, 2-Ethylbutyraldehyd, 2,2-Dimethylbutyraldehyd, 3,3-Dimethylbutyraldehyd, Caprylaldehyd, Caprinaldehyd.

Neben den genannten kurzkettigen Aldehyden sind insbesondere auch langkettige aliphatische Aldehyde geeignet, wie sie beispielsweise durch Oxosynthese aus linearen α-Olefinen erhalten werden können.

Besonders bevorzugt sind Enalisierungsprodukte, wie z.B. 2-Ethylhexenal, 2-Methylpentenal, 2,4-Diethyloctenal oder 2,4-Dimethylheptenal.

Bevorzugte Hydroxyaldehyde sind C₃-C₁₂-Hydroxyaldehyde, wie sie beispielsweise durch Aldolreaktion aus aliphatischen und cycloaliphatischen Aldehyden und Ketonen mit sich selbst oder Formaldehyd zugänglich sind. Beispiele sind 3-Hydroxypropanal, Dimethylolethanal, Trimethylolethanal (Pentaerythrital), 3-Hydroxybutanal (Acetaldol), 3-Hydroxy-2-ethylhexanal (Butyraldol), 3-Hydroxy-2-methylpentanal (Propionaldol), 2-Methylolpropanal, 2,2-Dimethylolpropanal, 3-Hydroxy-2-methyl-butanal, 3-Hydroxypentanal, 2-Methylolbutanal, 2,2-Dimethylolbutanal, Hydroxypivalinaldehyd. Besonders bevorzugt sind Hydroxypivalinaldehyd (HPA) und Dimethylolbutanal (DMB).

Bevorzugte Ketone sind Aceton, Butanon, 2-Pentanon, 3-Pentanon, 2-Hexanon, 3-Hexanon, Cyclohexanon, [sophoron, Methylisobutylketon, Mesityloxid, Acetophenon, Propiophenon, Benzophenon, Benzalaceton, Dibenzalaceton, Benzalacetophenon, 2,3-Butandion, 2,4-Pentandion, 2,5-Hexandion und Methylvinylketon.

Darüber hinaus können Carbonsäuren und Derivate davon, vorzugsweise solche mit 1-20 C-Atomen umgesetzt werden. Insbesondere sind die folgenden zu nennen:
Carbonsäuren, wie z.B. Ameisensäure, Essigsäure, Propionsäure, Buttersäure, Isobuttersäure, n-Valeriansäure, Trimethylessigsäure ("Pivalinsäure"), Capronsäure, Önanthsäure, Caprylsäure, Caprinsäure, Laurinsäure, Myristinsäure, Palmitinsäure, Stearinsäure, Acrylsäure, Methacrylsäure, Ölsäure, Elaidinsäure, Linolsäure, Linolensäure, Cyclohexancarbonsäure, Benzoesäure, Phenylessigsäure, o-Toluylsäure, m-Toluylsäure, p-Toluylsäure, o-Chlorbenzoesäure, p-Chlorbenzoesäure, o-Nitrobenzoesäure, p-Nitrobenzoesäure, Salicylsäure, p-Hydroxybenzoesäure, Anthranilsäure, p-Aminobenzoesäure, Oxalsäure, Malonsäure, Bernsteinsäure, Glutarsäure, Adipinsäure, Pimelinsäure, Korksäure, Azelainsäure, Sebacinsäure, Maleinsäure, Fumarsäure, 1,4-Cyclohexandicarbonsäure, Phthalsäure, Isophthalsäure, Terephthalsäure;
Carbonsäurehalogenide, wie z.B. die Chloride oder Bromide der oben genannten Carbonsäuren, insbesondere Acetylchlorid oder -bromid, Stearylchlorid oder -bromid und Benzoylchlorid oder -bromid, die insbesondere dehalogeniert werden;
Carbonsäureester, wie z.B. die C₁-C₁₀-Alkylester der oben genannten Carbonsäuren, insbesondere Methylformiat, Essigester, Buttersäurebutylester, Terephthalsäuredimethylester, Adipinsäuredimethylester, (Meth)acrylsäuremethylester, Butyrolacton, Caprolacton und Polycarbonsäureester, wie z.B. Polyacryl- und Polymethacrylsäureester und deren Copolymere und Polyester, wie z.B. Polymethylmethacrylat, wobei hier insbesondere Hydrogenolysen, also die Umsetzung von Estern zu den entsprechenden Säuren und Alkoholen, durchgeführt werden;
Carbonsäureanhydride, wie z.B. die Anhydride der oben genannten Carbonsäuren, insbesondere Essigsäureanhydrid, Propionsäureanhydrid, Benzoesäureanhydrid und Maleinsäureanhydrid.

Carbonsäureamide, wie z.B. Formamid, Acetamid, Propionamid, Stearamid, Terephthalsäureamid.

Ferner können auch Hydroxycarbonsäuren, wie z.B. Milch-, Äpfel-, Wein- oder Zitronensäure, oder Aminosäuren, wie z.B. Glycin, Alanin, Prolin und Arginin, umgesetzt werden.

Besonders bevorzugt wird das erfindungsgemäße Verfahren zur Hydrierung von Aldehyden und Hydroxyaldehyden eingesetzt.

Die zu hydrierende Carbonylverbindung kann dem Hydrierungsreaktor allein oder als Gemisch mit dem Produkt der Hydrierungsreaktion gasförmig oder flüssig zugeführt werden, wobei dies in unverdünnter Form oder unter Verwendung von zusätzlichem Lösungsmittel geschehen kann. Als zusätzliches Lösungsmittel eignen sich insbesondere Wasser, Alkohole wie Methanol, Ethanol und der Alkohol, der unter den Reaktionsbedingungen entsteht. Bevorzugte Lösungsmittel sind Wasser, THF, NMP, sowie Ether, wie z.B. Dimethyl-, Diethylether, MTBE, besonders bevorzugt ist Wasser.

Die Hydrierung sowohl in Sumpf- als auch in Rieselfahrweise führt man im allgemeinen bei einer Temperatur von 50 bis 250 °C, bevorzugt bei 70 bis 200 °C, besonders bevorzugt bei 100 bis 140 °C und einem Druck von 15 bis 250 bar, bevorzugt 20 bis 200 bar, besonders bevorzugt 25 bis 100 bar durch.

Mit dem erfindungsgemäßen Verfahren werden hohe Umsätze und Selektivitäten erzielt. Der eingesetzte Katalysator weist gegenüber den Katalysatoren nach dem Stand der Technik eine erhöhte Aktivität auf. Die Hydrierung kann daher - ohne Einbuße an Umsatz und Selektivität - mit deutlich höheren Katalysatorbelastungen durchgeführt werden. Ferner ist der Katalysator einfach herzustellen, da Schritte wie Formung und Calcinierung entfallen. Dadurch ist das erfindungsgemäße Verfahren besonders wirtschaftlich.

Die Erfindung wird durch die nachstehenden Beispiele näher erläutert.

### Beispiel 1: Hydrierung von Hydroxypivalinaldehyd (HPA) zu Neopentylglykol (NPG) in Rieselfahrweise

Als Ausgangslösung dient ein Gemisch aus 38 Gew.-% HPA und 38 Gew.-% NPG in 24 Gew.-% Wasser. Dieses Gemisch wurde in einem Reaktor von 50 cm Länge und 4,25 cm Innendurchmesser (Reaktorvolumen 200 ml), befüllt mit
a) 200 ml von nach EP-A-0 044 444 hergestelltem Al₂O₃-geträgertem Kupferkatalysator in Tablettenform (3 * 3 mm), Cu-Gehalt 36 Gew.-%, Schüttdichte 1090 g/l, BET-Oberfläche 101 m²/g, spezifische Cu-Oberfläche 11,5 m²/g als Katalysator A,
   beziehungsweise
b) 200 ml von nach WO 95/32171 hergestelltem SiO₂-geträgertem Kupferkatalysator in Kugelform (Durchmesser 3 mm), Cu-Gehalt 18 Gew.-%, Schüttdichte 605 g/l, BET-Oberfläche 212 m²/g, spezifische Cu-Oberfläche 9,8 m²/g, als Katalysator B,
   beziehungsweise
c) 200 ml von nach DE-A 44 46 907 hergestelltem Raney-Kupfer in Tablettenform (3 * 3 mm), Cu-Gehalt 72 Gew.-%, Schüttdichte 2000 g/l, Oberfläche nach Langmuir 24 m²/g, spezifische Cu-Oberfläche 2,6 m²/g, Hg-Porenvolumen 0,06 ml/g, mittlerer Porendurchmesser (aus Hg-Porosimetrie) 84 nm, als Katalysator C,
   beziehungsweise
d) 200 ml Raney-Kupfer in Form von Nuggets (Durchmesser 3 mm), Cu-Gehalt 56 Gew.-%, Schüttdichte 1950 g/l, Oberfläche nach Langmuir 26 m²/g, spezifische Cu-Oberfläche 3,1 m²/g, Hg-Porenvolumen 0,07 ml/g, mittlerer Porendurchmesser (aus Hg-Porosimetrie) 92 nm, als Katalysator D (erfindungsgemäß)
bei einer Katalysatorbelastung von 0,35 l HPA / l Katalysator * h bei 130 °C und 35 bar in Riesel- und Kreislauffahrweise (Kreislauf 9,5 l/h) hydriert.

### Beispiel 2: Hydrierung von 2,2-Dimethylolbutanal (DMB) zu 1,1,1-Trimethylolpropan in Sumpffahrweise

Als Ausgangslösung diente eine 45 gew.-%ige wäßrige DMB-Lösung. Diese Lösung wurde in einem Reaktor von 30 cm Länge und 2,5 cm Innendurchmesser, befüllt mit 150 ml des Katalysators B, C beziehungsweise D (erfindungsgemäß) aus Beispiel 1 bei 120 °C und 90 bar in Sumpffahrweise hydriert. Die Lösung wurde in geradem Durchgang durch den Reaktor gepumpt, wobei mit verschiedenen Katalysatorbelastungen: 0,2, 0,3, 0,4 und 0,6 kg DMB / l Katalysator * h gearbeitet wurde.

### Tabelle 1 faßt die Ergebnisse zusammen.

Die Ergebnisse verdeutlichen, daß mit dem Katalysator D unter sonst gleichen Bedingungen die höchsten Umsätze und Selektivitäten erreicht werden.

**Tabelle 1**

| **Katalysator** | **Edukt** | **Verfahrensweise** | **Katalysator- belastung** **kg/l**_{**KAT**}**·h** | **Umsatz (aus den GC-Flächen-%)** **%** | **Selektivität** **(aus den GC- Flächen-%)** **%** |
|---|---|---|---|---|---|
| A | HPA | Riesel/ Kreislauf | 0,35 | 86,8 | 89,1 |
| B | HPA | Riesel/ Kreislauf | 0,35 | 94 | 92,5 |
| B | DMB | Sumpf | 0,2 | 99,44 | 78,91 |
| B | DMB | Sumpf | 0,3 | 90,84 | 75,28 |
| C | HPA | Riesel/ Kreislauf | 0,35* | 95,2 | 93,3 |
| C | DMB | Sumpf | 0,2 | 100 | 89,97 |
| C | DMB | Sumpf | 0,4 | 97,65 | 87,34 |
| C | DMB | Sumpf | 0,6 | 88,79 | 74,52 |
| D | HPA | Riesel/ Kreislauf | 0,35 | 96,8 | 94,2 |
| D | DMB | Sumpf | 0,2 | 100 | 91,17 |
| D | DMB | Sumpf | 0,4 | 100 | 90,7 |
| D | DMB | Sumpf | 0,6 | 95,45 | 87,3 |

| | | | | | |
|---|---|---|---|---|---|
| *l_{HPA}/kg_{Kat}·h | | | | | |

## Patentansprüche

1. Verfahren zur katalytischen Hydrierung einer Carbonylverbindung oder eines Gemisches aus zwei oder mehr Carbonylverbindungen in Gegenwart eines Raney-Kupfer-Katalysators, **dadurch gekennzeichnet, daß** der Raney-Kupfer-Katalysator in Form von Nuggets eingesetzt wird, wobei die Nuggets Körner unregelmäßiger Geometrie mit einer Korngröße von 0,5 bis 10 mm sind.

2. Verfahren nach Anspruch 1, **dadurch gekennzeichnet, daß** der Raney-Kupfer-Katalysator folgende Eigenschaften aufweist:
- Korngröße der Nuggets 2 bis 7 mm
- Kupfer-Gehalt 40 bis 90 Gew.-%
- Oberfläche nach Langmuir 5 bis 50 m²/g
- Cu-Oberfläche 0,5 bis 7 m²/g
- Hg-Porenvolumen 0,01 bis 0,12 ml/g
- mittlerer Porendurchmesser 50 bis 300 nm

3. Verfahren nach Anspruch 1 oder 2, **dadurch gekennzeichnet, daß** die katalytische Hydrierung als Festbettreaktion in Rieselfahrweise durchgeführt wird.

4. Verfahren nach Anspruch 1 oder 2, **dadurch gekennzeichnet, daß** die katalytische Hydrierung als Festbettreaktion in Sumpffahrweise durchgeführt wird.

5. Verfahren nach Anspruch 3 oder 4, **dadurch gekennzeichnet, daß** das Verfahren zusätzlich in Kreislauffahrweise durchgeführt wird.

6. Verfahren nach einem der Ansprüche 3 bis 5, **dadurch gekennzeichnet, daß** man die Hydrierung bei einer Temperatur von 70 bis 200 °C und einem Druck von 20 bis 200 bar durchführt.

7. Verfahren nach einem der Ansprüche 1 bis 6, **dadurch gekennzeichnet, daß** als Carbonylverbindung ein aliphatischer Aldehyd oder ein aliphatischer Hydroxyaldehyd oder ein Gemisch aus zwei oder mehr davon eingesetzt wird.

8. Verfahren nach Anspruch 7, **dadurch gekennzeichnet, daß** als Carbonylverbindung Hydroxypivalinaldehyd oder Dimethylolbutanal eingesetzt wird.

9. Verfahren nach einem der Ansprüche 1 bis 8, **dadurch gekennzeichnet, daß** in Gegenwart von Wasser als Lösungsmittel gearbeitet wird.

## Claims

1. A process for the catalytic hydrogenation of a carbonyl compound or of a mixture of two or more carbonyl compounds in the presence of a Raney copper catalyst, wherein the Raney copper catalyst is employed in the form of nuggets, the nuggets being particles of irregular geometry having a particle size of from 0.5 to 10 mm.

2. A process as claimed in claim 1, wherein the Raney copper catalyst has the following properties:
- size of the nuggets from 2 to 7 mm
- copper content from 40 to 90% by weight
- Langmuir surface area from 5 to 50 m²/g
- Cu surface area from 0.5 to 7 m²/g
- Hg pore volume from 0.01 to 0.12 ml/g
- average pore diameter from 50 to 300 nm.

3. A process as claimed in claim 1 or 2, wherein the catalytic hydrogenation is carried out as fixed bed reaction in a downflow procedure.

4. A process as claimed in claim 1 or 2, wherein the catalytic hydrogenation is carried out as fixed bed reaction in an upflow procedure.

5. A process as claimed in claim 3 or 4, wherein the process is additionally carried out in a recycle procedure.

6. A process as claimed in any of claims 3 to 5, wherein the hydrogenation is carried out at from 70 to 200°C under a pressure of from 20 to 200 bar.

7. A process as claimed in any of claims 1 to 6, wherein an aliphatic aldehyde or an aliphatic hydroxy aldehyde or a mixture of two or more thereof is employed as carbonyl compound.

8. A process as claimed in claim 7, wherein hydroxypivalaldehyde or dimethylolbutanal is employed as carbonyl compound.

9. A process as claimed in any of claims 1 to 8, wherein water is present as solvent.

## Revendications

1. Procédé pour l'hydrogénation catalytique d'une combinaison carbonyle ou d'un mélange de deux combinaisons carbonyle ou plus, en présence d'un catalyseur de Raney en cuivre, **caractérisé en ce que** le catalyseur de Raney en cuivre est utilisé sous forme de nuggets, dans lequel les grains des nuggets sont d'une géométrie irrégulière avec une granulométrie de 0,5 à 10 mm.

2. Procédé selon la revendication 1, **caractérisé en ce que** le catalyseur de Raney en cuivre présente les caractéristiques suivantes :
- granulométrie des nuggets 2 à 7 mm
- teneur en cuivre 40 à 90 % du poids
- surface selon Langmuir 5 à 50 m²/g
- surface en Cu 0,5 à 7 m²/g
- volume poreux de Hg 0,01 à 0,12 ml/g
- diamètre moyen des pores 50 à 300 nm

3. Procédé selon la revendication 1 ou 2, **caractérisé en ce que** l'hydrogénation catalytique est réalisée en tant que réaction à lit fixe en fonctionnement par ruissellement.

4. Procédé selon la revendication 1 ou 2, **caractérisé en ce que** l'hydrogénation catalytique est réalisée en tant que réaction à lit fixe en fonctionnement par sédimentation.

5. Procédé selon la revendication 3 ou 4, **caractérisé en ce que** le procédé est réalisé en plus en fonctionnement en circuit.

6. Procédé selon l'une des revendications 3 à 5, **caractérisé en ce que** l'on réalise l'hydrogénation à une température de 70 à 200°C et à une pression de 20 à 200 bar.

7. Procédé selon l'une des revendications 1 à 6, **caractérisé en ce qu'**un aldéhyde aliphatique ou un hydroxylaldéhyde aliphatique ou un mélange de deux ou plus de ceux-ci sont utilisés comme combinaison carbonyle.

8. Procédé selon la revendication 7, **caractérisé en ce que** de l'hydroxypivalinaldéhde ou du diméthylolbutanal est utilisé comme combinaison carbonyle.

9. Procédé selon l'une des revendications 1 à 8, **caractérisé en ce que** le travail est effectué en présence d'eau en tant que solvant.
